# EUROPEAN PATENT SPECIFICATION

(11) **EP 3 257 433 B1**
(45) Date of publication and mention of the grant of the patent: **09.08.2023**
(21) Application number: 17174304.0
(22) Date of filing: 02.06.2017
(51) Int. Cl.: A61B 3/00, A61B 3/15, G06T 5/50, A61B 3/117, A61B 3/10, A61B 3/12

(54) **OPHTHALMIC IMAGING DEVICE AND GENERATION METHOD OF OPHTHALMIC SYNTHETIC IMAGE**
OPHTHALMISCHE ABBILDUNGSVORRICHTUNG UND ERZEUGUNGSVERFAHREN FÜR OPHTHALMISCHE SYNTHETISCHE BILDER
DISPOSITIF D'IMAGERIE OPHTALMIQUE ET PROCÉDÉ DE PRODUCTION D'IMAGE SYNTHÉTIQUE OPHTALMIQUE

(30) Priority: 03.06.2016 JP 2016111746
(43) Date of publication of application: 20.12.2017
(73) Proprietor: Nidek co., Ltd., Gamagori Aichi (JP)
(72) Inventor: FUJIU, Kenshiro, Aichi (JP); MURASE, Yuji, Aichi (JP)
(74) Representative: Hoefer & Partner Patentanwälte mbB

(56) References cited:
- JP-A- 2009 160 190
- US-A1- 2010 097 573
- US-A1- 2011 234 977
- US-B1- 6 829 383

## Description

### BACKGROUND

The present disclosure relates to an ophthalmic imaging device and a generation method of an ophthalmic synthetic image.

In the related art, an ophthalmic imaging device is known which captures a front image of a subject's eye. For example, as disclosed in JP-A-2016-28687, a device is known in which a lens system (refraction system) is applied to an objective optical system of a fundus imaging device which is a type of an ophthalmic imaging device, and in which imaging is performed via the objective optical system including the lens system in both ways of irradiating a fundus with illumination light and receiving fundus reflected light.

Here, in the device where the lens system is applied to the objective optical system, if the front image of the subject's eye is needed, an objective lens reflected image obtained by the reflection on a lens surface of the objective optical system is included in a captured image in some cases.
US 2010/0097573 A1 discloses a slit lamp mounted eye imaging device for viewing wide field and/or magnified views of the retina or the anterior segment through an undilated or dilated pupil. The apparatus images posterior and anterior segments of the eye, and sections/focal planes in between and contains an illumination system that uses one or more LEDs, shifting optical elements, and/or aperture stops where the light can be delivered into the optical system on optical axis or off axis from center of optical system and return imaging path from the retina, thereby creating artifacts in different locations on retina. Image processing is employed to detect and eliminate artifacts from images. The device is well suited for retinal imaging through an undilated pupil, non-pharmacologically dilated, or a pupil as small as 2 mm.; Two or more images with reflection artifacts can be created and subsequently recombined through image processing into a composite artifact-free image.
US 6 829 383 B1 discloses a computer-implemented method and system for equalizing a brightness, contrast and color of two or more images of an eye fundus. Because left and right images are taken from different camera positions, or because two images have been taken at different times, the resulting images have different brightness, contrast and color. Groups are formed from the pixels making up the images. The groups can be straight lines oriented perpendicularly to the direction of camera motion. The intensity of each pixel in a group is measured, and the group's mean and variance are then calculated. The intensity of the groups are then adjusted in order that a group from the left image will have the same mean intensity and variance as the corresponding group from the right image. The measurements and adjustments can be made for each of the red, green and blue intensities of each pixel.
JP 2009 160 190 A discloses an ophthalmic imaging apparatus that has an irradiation means to irradiate an examined eye with light from a light source issuing light flux with a low coherent length, a scanning means to scan the measurement light, an optical path difference change means to change the optical path difference between reference light and measurement light irradiating the examined eye, an interference optical system to obtain the tomogram image of the examined eye by receiving interference light obtained through the integration of the reflective light of the reference and measurement light, and a display means to display the tomogram image. The apparatus is also provided with a position specifying means to specify an arbitrary point on the tomogram image displayed on the display means and a control means to change the optical path differences by the optical path difference change means from the information of specified position or of its movement from the position specifying means, and to thereby change the depth position of the examined eye relative to the optical path of reference light.
US 2011 234 977 A1 discloses a slit lamp mounted eye imaging, a slit lamp integrated, a handheld, OCT integrated, or attached to a separate chinrest-joystick assembly apparatus and method for producing a wide field and/or magnified views of the posterior or the anterior segments of an eye through an undilated or dilated pupil. The apparatus images sections and focal planes and utilizes an illumination system that uses one or more LEDs, shifting optical elements, flipping masks, and/or aperture stops where the light can be delivered into the optical system on optical axis or off axis from center of optical system and return imaging path from the eye, creating artifacts in different locations on the eye image. Image processing is employed to detect and eliminate artifacts and masks from images.

### SUMMARY

The present disclosure is made in view of the above-described problem in the related art, and a technical object is to provide an ophthalmic imaging device and a generation method of an ophthalmic synthetic image, which can satisfactorily obtain a front image of a subject's eye.

The solution of this object is achieved by the independent claims. The dependent claims contain advantageous embodiments of the present invention.

According to the present disclosure, it is possible to satisfactorily obtain a front image of a subject's eye.

### BRIEF DESCRIPTION OF THE DRAWINGS

Fig. 1 is a view schematically illustrating a process when a synthetic front image is formed using a first synthesis method.
Fig. 2 is a view schematically illustrating a process when a synthetic front image is formed using a second synthesis method.
Fig. 3 is a view illustrating luminance distribution in a mask region used for the second synthesis method.
Fig. 4 is a view illustrating a method in which an image in a wider range than that of an original image is formed as the synthetic front image.
Fig. 5 is a view illustrating a specific example of an output aspect of guide information.
Fig. 6 is a view illustrating a case where defective macular ring reflex appears on the synthetic front image.
Fig. 7 is a view illustrating an external configuration of SLO according to an example.
Fig. 8 is a view illustrating an optical system in SLO.
Fig. 9 is a view illustrating an example of a control system in SLO.
Fig. 10 is a flowchart illustrating an operation flow according to an example.

### DESCRIPTION OF ILLUSTRATIVE EMBODIMENTS

Hereinafter, an ophthalmic imaging device and an embodiment of a generation method of an ophthalmic synthetic image will be described with reference to the drawings. The ophthalmic imaging device may be used with a computer which generates the ophthalmic synthetic image by executing an image processing program.

The ophthalmic imaging device acquires a front image (objective lens reflected image) of a subject's eye. For example, the ophthalmic imaging device may acquire the front image of a fundus or may acquire the front image of an anterior ocular segment. In the present disclosure, the ophthalmic imaging device may have an imaging optical system, an image acquisition unit (image generation unit), a position adjustment unit, and an image processing unit.

### <Imaging Optical System>

The imaging optical system is a primary optical system used in order to obtain the front image of a fundus image. The imaging optical system includes an irradiation optical system and a light receiving optical system. The irradiation optical system irradiates the subject's eye with illumination light emitted from a light source via an objective lens system. The light receiving optical system has a light receiving element which receives reflected light of the illumination light reflected on the subject's eye via the objective lens system.

According to the invetion, the ophthalmic imaging device is a SLO, the irradiation optical system includes an optical scanner. The subject's eye is irradiated with the illumination light (laser light) emitted from the light source, and the optical scanner scans a tissue of the subject's eye with the laser light.

The objective lens system includes at least one lens. The objective lens system may include only a refractive element such as the lens, and may include a reflective element such as a mirror in addition to the lens.

### <Image Acquisition Unit>

The image acquisition unit (image generation unit) acquires (generates) the front image of the subject's eye, based on a signal transmitted from the light receiving element. For example, in the SLO, every time the optical scanner performs scanning with the illumination light of one frame, a light receiving signal of one frame output from the light receiving element is processed by the image acquisition unit. As a result, the front image of one frame is generated and acquired.

The front image obtained by the image acquisition unit may be a captured image or an observation image. For example, the captured image may be a still image to be stored in a memory based on a release. For example, release timing may be timing for a predetermined operation to be performed by an examiner, or may be timing regulated in advance in an imaging program. The observation image is the front image acquired at any time based on the signal transmitted from the light receiving element and displayed on a monitor as a moving image. The observation image and the captured image may be images in which light having mutually different wavelength ranges is used as the illumination light.

In the front image obtained by the image acquisition unit, an objective lens reflected image of the illumination light due to the objective lens system may appear as one type of artifacts. Hereinafter, the objective lens reflected image due to this objective lens system is referred to as an "objective lens reflected image".

### <Position Adjustment Unit>

The position adjustment unit is used in order to adjust a position where the objective lens reflected image is formed in the front image so as to be aligned with a tissue (for example, either the anterior ocular segment or the fundus) of the subject's eye in the front image, by changing a position relationship between an optical axis of the imaging optical system and a visual axis of the subject's eye. If the position relationship is changed between the optical axis of the imaging optical system and the visual axis, the position relationship is changed between the tissue of the subject's eye in the front image of the subject's eye and the optical axis of the imaging optical system. On the other hand, the position of the objective lens reflected image in the front image is constant with respect to the optical axis of the imaging optical system regardless of the position relationship between the optical axis of the imaging optical system and the visual axis. Therefore, in accordance with the position relationship between the optical axis of the imaging optical system and the visual axis, the position of the objective lens reflected image in the front image is changed with respect to the tissue of the subject's eye.

The position adjustment unit may include a fixation optical system, may include an imaging optical system drive mechanism, or may include both of these. In the fundus imaging device, the position adjustment unit adjusts an angle between an orientation of the optical axis of the imaging optical system and an orientation of the visual axis of the subject's eye.

The fixation optical system induces fixation by presenting a fixation target to the subject's eye. The fixation optical system may be an internal fixation optical system or an external fixation optical system (external fixation lamp). Alternatively, the fixation optical system may include both of these. The internal fixation optical system is disposed inside a housing of the device, and presents the fixation target to the subject's eye. The external fixation optical system is disposed outside the housing, and presents the fixation target to an eye laterally opposite to the subject's eye. The fixation optical system may be capable of switching a presentation position of the fixation target to a plurality of positions in a direction intersecting the optical axis of the imaging optical system. A fixation lamp may be used as the fixation target in the fixation optical system, or a screen of a display such as an LCD may be used. In addition, the fixation optical system may be shared with the irradiation optical system. For example, visible light intermittently blinking from the light source may be used as a fixation luminous flux (in other words, the fixation target). Details will be described later.

The imaging optical system drive mechanism changes a position relationship between the visual axis of the subject's eye E and the optical axis of the imaging optical system (also referred to as an imaging optical axis). In the fundus imaging device, the imaging optical system drive mechanism may cause the imaging optical system to rise or pivot based on the subject's eye. The drive mechanism may include an actuator for displacing the imaging optical system, based on a control signal. In a configuration in which the imaging optical system is displaced by the imaging optical system drive mechanism, it is desirable that the presentation position of the fixation target is located at a constant position with respect to a space even when the imaging optical system is displaced. For example, a fixation target presentation unit may be controlled in conjunction with the actuator. In this case, the presentation position of the fixation target may be displaced in a phase opposite to the displacement of the imaging optical system. A configuration may be adopted which is provided independently of the imaging optical system so that fixation is induced by the external fixation lamp fixedly disposed with respect to the space.

### <Image Processing Unit>

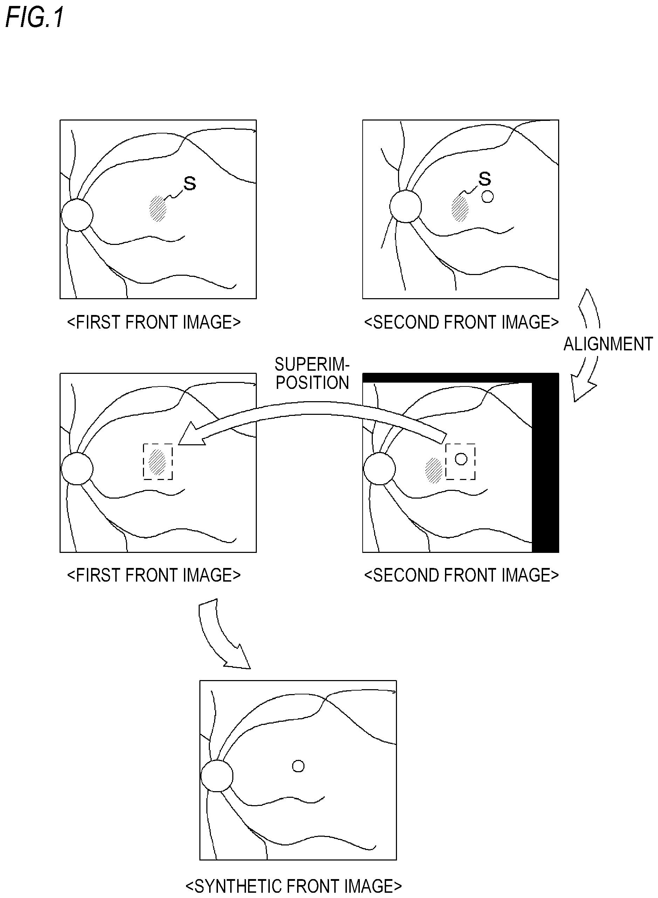

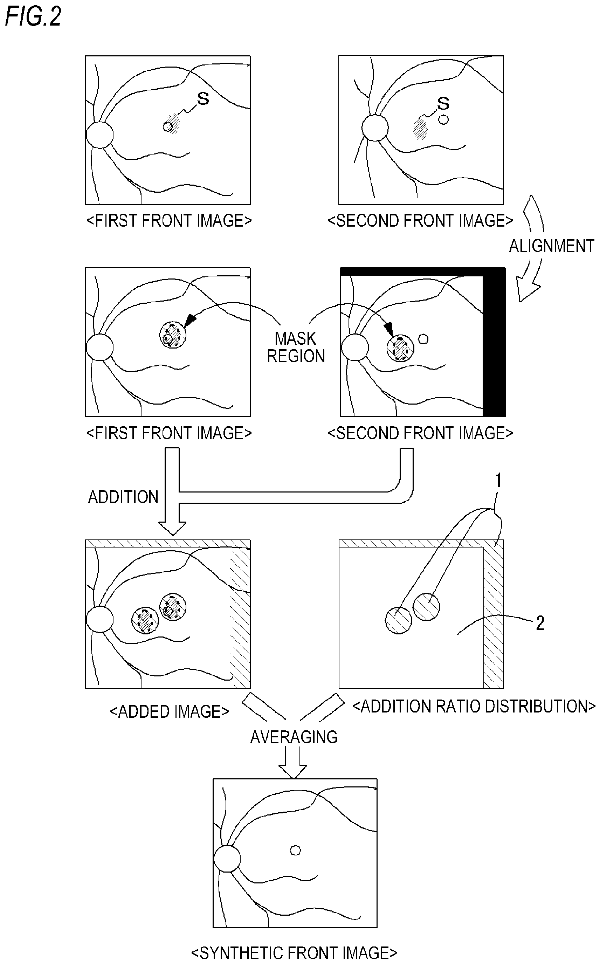

The image processing unit ([image processing step]) obtains a synthetic front image, based on at least two of a first front image and a second front image (refer to Figs. 1 and 2). The synthetic front image is one type of ophthalmic synthetic images. The synthetic front image may be obtained by synthesizing at least two of the first front image and the second front image. Both the first front image and the second front image are obtained by the image acquisition unit. In the second front image, a position of the objective lens reflected image with respect to the tissue of the subject's eye is different from that of the first front image, and the second front image includes a region where the objective lens reflected image is located in the first front image. The objective lens reflected image is represented by a reference symbol S in Figs. 1 and 2. The second front image has information on the tissue of the subject's eye in the region where the objective lens reflected image is formed in the first front image. Therefore, the region where the objective lens reflected image is included in the first front image can be supplemented by an image region included in the second front image, which has the same position relationship with respect to the tissue of the subject's eye as that of the region. As a result, the objective lens reflected image can be restrained in the synthetic front image. In other words, the term "synthesis" between the first front image and the second front image may mean that "the region including the objective lens reflected image in the first front image is supplemented by the image region included in the second front image, which has the same position relationship with respect to the tissue of the subject's eye as that of the region". The synthetic front image may be obtained as a result of synthesizing a plurality of the front images including at least the first front image and the second front image. That is, in addition to the first front image and the second front image, the synthetic front image may be formed by further synthesizing one or more front images. The "region including the objective lens reflected image in the first front image" may be a region including a main pixel affected by object reflection. For example, the region may be a region having a magnitude of approximately 2σ (σ = standard deviation) of luminance distribution in the objective lens reflected image. Alternatively, the magnitude may be greater or smaller.

The first front image and the second front image may be images in which the imaging ranges are substantially in the same relationship. Here, a case where both of these are substantially in the same relationship includes not only a case where the imaging ranges of the first front image and the second front image are completely the same as each other but also a case where both the imaging ranges include deviation. For example, a case where an area of a superimposed portion between the first front image and the second front image is equal to or larger than half of an area of each image may be included in the case where both of these are substantially in the same relationship. The first front image and the second front image may be the front image obtained at the same depth in the subject's eye. The first front image and the second front image may be the front image obtained at the mutually different depths in the subject's eye. For example, in a case of the fundus imaging device, each front image may be an image of a retinal surface. In a case where the present disclosure is applied to an anterior ocular segment camera, each front image may be an image of an anterior ocular segment surface.

When the images are synthesized, alignment (image registration) between the first front image and the second front image may be performed by the image processing unit. The alignment described herein may include at least any one of parallel movement, rotation, enlargement/reduction, affine transformation, and nonlinear deformation. For example, in the alignment, any one may be used from position information of characteristic sites (for example, in the fundus front image, any one of a blood vessel, a macula, and a papilla) of the subject's eye in the front image and phase information of the front image. A deviation amount between the first front image and the second front image may be a known value, in some cases. For example, the deviation amount is a value depending on the displacement amount of the position relationship between the optical axis of the imaging optical system and the visual axis, between the time of capturing the first front image and the time of capturing the second front image. This displacement amount may be a predetermined value. The displacement amount of the position relationship may be the amount detected by a sensor. The image processing unit may perform the alignment between the first front image and the second front image by using the deviation amount based on these values.

When the first front image and the second front image are aligned with each other, it is preferable that both of these are in a relationship where the objective lens reflected images are completely separated from each other. However, the present disclosure is not necessarily limited thereto. According to the above-described alignment, even in a case where the objective lens reflected images partially overlap each other, at least a portion of the region where the objective lens reflected image is formed in the first front image can be supplemented by the second front image. Accordingly, in the synthetic front image, the objective lens reflected image is reduced compared to the original image (that is, the first front image or the second front image).

The front image includes distortion caused by the optical system. This distortion increases toward a peripheral portion of the image. Information (distortion information) indicating a degree of the distortion in each region of the image can be acquired in advance as a design value or as a result of calibration. Based on the distortion information, the image processing unit may correct the distortion in each of the front images, and then may perform the above-described alignment and synthesizing process. In this manner, the synthetic front image having satisfactory image quality is likely to be formed.

### <Synthesis Using Replacement>

For example, the region (replacement target region) including the objective lens reflected image in the first front image may be replaced with the image region of a portion of the second front image so that the first front image and the second front image are "synthesized (or "supplemented)" (refer to Fig. 1). The portion of the second front image which is used for replacement may be the image region where the position with respect to the tissue of the subject's eye is the same as that of the replacement target region in the first front image. In this case, the first front image in which the objective lens reflected image is partially or entirely replaced with the tissue of the subject's eye is obtained as the synthetic front image.

The image processing unit may detect the objective lens reflected image from the first front image by means of image processing so as to specify the replacement target region in accordance with a detection result. In the front image, the objective lens reflected image mainly appears in the vicinity of the optical axis of the imaging optical system. Accordingly, the replacement target region in the first front image may be set in advance in a prescribed range in the vicinity of the optical axis. In the second front image, the image region to be replaced with the replacement target region of the first front image may be obtained as follows, for example. That is, the image processing unit may associate position information of the first front image with position information of the second front image by aligning the first front image and the second front image with each other. In this manner, a location associated with the replacement target region of the first front image may be obtained on the second front image. However, the present disclosure is not necessarily limited to this method.

The image processing unit may set a size of the replacement target region in the first front image and a size of the image region to be replaced with the replacement target region in the second front image, in accordance with a size of the objective lens reflected image. Here, the size of the objective lens reflected image varies depending on imaging conditions such as the amount of diopter adjustment, a wavelength range of the illumination light (for example, the laser light), an output of the illumination light, and gain. In view of any one of these imaging conditions, the size of the replacement target region and the size of the image region to be replaced with the replacement target region in the second front image may be set. For example, a function or a look-up table which indicates a relationship between the size of the objective lens reflected image and the imaging condition may be prepared in advance based on a calibration result, and may be stored in a memory of the device. The size of the objective lens reflected image (or the size of the replacement target region) may be acquired with reference to the function or the look-up table, based on the imaging condition when the first front image and the second front image are acquired. The size of the objective lens reflected image may be detected by performing image processing on either the first front image or the second front image.

The image processing unit is configured to perform a process (for example, a cross fade process) for smoothing a luminance change in a joining portion between the first front image and the second front image, when the first front image and the second front image are "synthesized".

### <Synthesis Using Addition (Mainly, Averaging)>

For example, the process of "synthesizing (or "supplementing")" the first front image and the second front image by the image processing unit may include a process of adding the first front image and the second front image (refer to Fig. 2). It is conceivable that a luminance value is saturated if the images are simply added to each other. Accordingly, an averaging process of decreasing the luminance value in accordance with the number of images used for the addition may be appropriately performed subsequently to the addition process. In other words, the synthetic front image may be generated by performing an averaging process on a plurality of the front images including at least the first front image and the second front image.

Here, in a case where the synthetic front image is obtained by simply adding (or averaging) the first front image and the second front image, there is a possibility that a location of the objective lens reflected image in the first front image and the second front image may be expressed with greatly different luminance compared to the surroundings in the synthetic front image. Therefore, for example, image processing means may multiply the region including the objective lens reflected image in one or both of the first front image and the second front image by a mask region, and then may perform the addition process (or the averaging process).

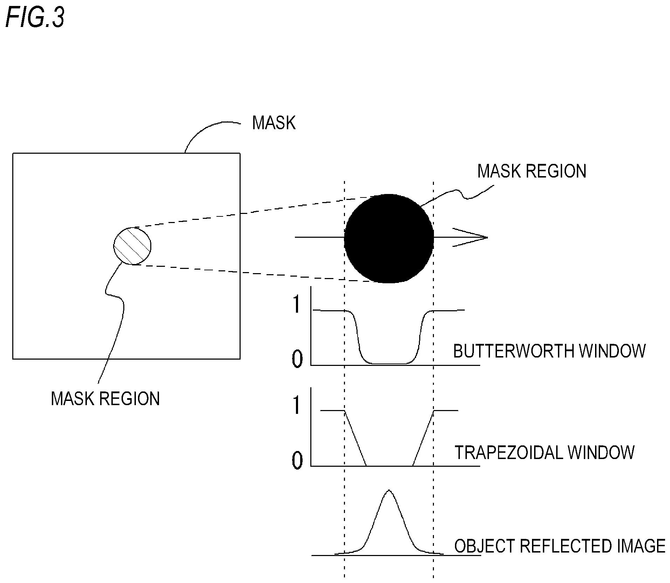

The region including the objective lens reflected image is multiplied by the mask region. In this manner, luminance distribution in the region including the objective lens reflected image is flattened by background luminance. For example, the background luminance may be the lowest luminance value if the image has a black background, or may be the highest luminance value if the image has a white background. The mask region superimposed on the objective lens reflected image may be formed using a two-dimensional window function having a flat portion. For example, the total length of the flat portion may be approximately 2σ (σ = standard deviation) of the luminance distribution of the objective lens reflected image. Alternatively, the total length may be greater or smaller. It is preferable that the two-dimensional window function has a shape which continuously varies from the outside of a section to the flat portion. A type of this two-dimensional window function may be appropriately selected. For example, as illustrated in Fig. 3, a Butterworth window may be used. In addition, a trapezoidal window may be used. Although details will be described later, since the two-dimensional window function has the shape continuously varying from the outside of the section to the flat portion, the location of the objective lens reflected image in the first front image and the second front image is less likely to be noticeable in the synthetic front image. A size or a shape of a mask superimposed on the objective lens reflected image may be fixed, or may be adjusted in accordance with the size or the shape of the objective lens reflected image. The size or the shape of the objective lens reflected image may be directly detected from the front image, or may be estimated based on the above-described imaging conditions.

In a case where the superimposed portion of the first front image and the second front image is simply added (simply averaged) after mask processing, the location multiplied by the mask region becomes noticeable in the added image in some cases. Therefore, for example, the image processing unit may obtain the synthetic front image in such a way that the first front image and the second front image are added using different addition ratios for each region (in other words, by averaging). In the superimposed portion of at least the first front image and the second front image, mutually different addition ratios are set for the mask region (the region multiplied by the mask region) and the surroundings.

For example, the addition ratio may be a value indicating weighted averaging for each region. As an example, the addition ratio illustrated in the addition ratio distribution in Fig. 2 is a value obtained by dividing the luminance value of a simply added image of the first front image and the second front image after the mask region is multiplied. The value obtained after dividing is the luminance value of the synthetic front image.

For example, the addition ratio distribution is obtained by adding the masks to each other in the first front image and the second front image in the same position relationship as that of the simply added image. In this case, the value of the addition ratio is "1" in the flat portion of the mask region with regard to the superimposed portion of the first front image and the second front image in the simply added image, and is "2" outside the section of the mask region. The value is between "2" and "1" in a transition portion from the outside of the section of the mask region to the flat portion. In addition, the addition ratio with regard to a portion where the first front image and the second front image are not superimposed on each other is "1".

However, the averaging method is not necessarily limited to the example in Fig. 2. For example, the synthetic front image may be generated by respectively weighting the luminance value in the first front image and the luminance value in the second front image and then performing the addition.

As a result of averaging (weighted averaging) the first front image and the second front image as described above, the synthetic front image is obtained where a trace of the mask region is less likely to be noticeable. The value of the above-described addition ratio is merely an example in a case of averaging (weighted averaging) two front images. The value can be appropriately changed in accordance with the number of the front images to be synthesized and characteristics of the two-dimensional window function in the mask region.

However, as described above, the process of multiplying the mask region (mask process) and averaging the regions with the addition ratio different from that of the other superimposed portion can obtain the synthetic front image by the addition, but is not absolutely essential.

For example, adding the front images whose positions of the objective lens reflected image with respect to the tissue of the subject's eye in the image are different from each other by using more front images is a useful technique in restraining the objective lens reflected image. In a case of the addition using three or more front images whose positions of the objective lens reflected image with respect to the tissue of the subject's eye in the image are different from each other, a noise reduction effect (for example, a reduction effect of random noise whose generated position and intensity are changed in each image) is expected at the formation position of the objective lens reflected image.

In a case where the synthetic front image is generated by the addition, it is not necessary to add all of the first front images and the second front images. Only a portion of one front image between the first front image and the second front image may be cut out so that the cutout range is added to the other front image. The cutout range in one front image includes the tissue where the objective lens reflected image is located in at least the other front image.

### <Reduction of Luminance Difference between Front Images>

The image processing unit may perform processing for restraining a luminance difference between the images on at least the location used for the synthesis of the first front image and the second front image, and then, may synthesize both of these. Specifically, contrast, brightness, or both of these in at least one of the images may be adjusted. In this manner, the luminance change in a joining portion between the first front image and the second front image is restrained. Accordingly, the joining portion is less likely to be noticeable.

### <Generation of Wide Range Front Image>

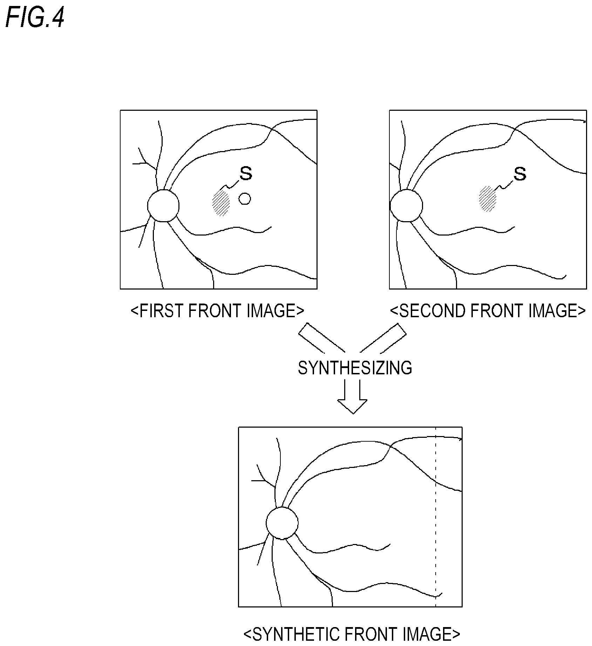

The image processing unit may form the synthetic front image as an image having a wider range than any one of the first front image and the second front image by using not only the superimposed portion between the first front image and the second front image but also the non-superimposed portion (refer to Fig. 4). As described above, the distortion caused by the optical system increases toward the edge of the image. Accordingly, for example, the images may be synthesized in a state where a prescribed region corresponding to the edge of each front image is removed. When the above-described wide range synthetic front image is formed, the method of synthesizing the front images in order to restrain the objective lens reflected image may be a method using the addition, or may be a method using the replacement. For example, in a case of the method using the replacement, the synthesizing process of the first front image and the second front image by the image processing unit may include a process in which the image region where the first front image and the second front image are not superimposed on each other is joined to one front image whose region including the objective lens reflected image is replaced.

### <Acquisition Operation of Front Image>

The control unit which controls an acquisition operation of the front image may be disposed in the ophthalmic imaging device. The control unit may control the operation of the device in an [imaging step]. In the [imaging step], the ophthalmic imaging device captures a plurality of the front images whose positions of the reflected images of the illumination light emitted by the objective lens system are different from each other with respect to the tissue of the subject's eye. Herein, a plurality of the front images include the first front image and the second front image. A plurality of the front images captured in the [imaging step] are stored in a memory ([front image acquisition step]).

In order to obtain at least one of the first front image and the second front image, the control unit may adjust the position relationship between the optical axis of the imaging optical system and the visual axis of the subject's eye by controlling the position adjustment unit. The control unit may output guide information. As a matter of course, both of these may be used in combination. The adjustment control of the position relationship or the guide information may be prepared so as to displace the image captured as the first front image and the second front image to the position relationship where the positions having the objective lens reflected image are not superimposed on each other. For example, the guide information may guide an examiner or a subject to perform an operation for adjusting the position relationship between the optical axis of the imaging optical system and the visual axis of the subject's eye. For example, the guide information may be operation guidance for the examiner. The guide information may be information for guiding a change in the fixation position to the subject. For example, this guide information may be sound information output from a speaker, or graphical information (for example, character information or figure information, details will be described later) displayed on a monitor. Alternatively, the guide information may be information delivered to other examiners or the subject.

An imaging sequence for at least capturing the first front image and the second front image is predetermined, and the imaging sequence is executed by the control unit. In this manner, the first front image and the second front image may be captured and acquired.

For example, at least one of the following [first imaging sequence] and [second imaging sequence] may be determined in advance.

[First Imaging Sequence] The first front image and the second front image are respectively acquired based on an individual release operation.

[Second Imaging Sequence] The first front image and the second front image are automatically and continuously acquired.

In the first imaging sequence or the second imaging sequence, first, one of the first front image and the second front image is acquired (captured). Thereafter, the control unit may automatically adjust the position relationship (more specifically, adjustment of the position relationship between the optical axis of the imaging optical system and the visual axis of the subject's eye) to the other imaging position. Then, after the position relationship is adjusted, the remaining one (the other one) of the first front image and the second front image may be automatically or manually acquired in accordance with a type of the imaging sequence. As a matter of course, after one of the first front image and the second front image is acquired (captured) in the first imaging sequence or the second the imaging sequence, the position relationship may be manually adjusted to the other imaging position. In this case, an output of the guide information may be controlled.

The adjustment control of the position relationship between the optical axis of the imaging optical system and the visual axis of the subject's eye, or the output control of the guide information may start, for example, by being triggered when at least any one of the first front image and the second front image is captured. As a matter of course, both of these may be performed before any front image is acquired.

In a case where the adjustment of the position relationship between the optical axis of the imaging optical system and the visual axis of the subject's eye is controlled, the control unit may capture a plurality of the front images including at least either the first front image or the second front image while switching the position relationship between the optical axis of the imaging optical system and the visual axis of the subject's eye by controlling the position adjustment unit. For example, the control unit may drive the position adjustment unit in a predetermined drive range by being triggered when a trigger signal is input based on the release operation. While the position adjustment unit is driven, the control unit may capture (in other words, may cause the image acquisition unit to acquire) a plurality of the front images used for the synthetic front image. In this case, for example, the control unit may displace the presentation position of the fixation target or the imaging optical system between a predetermined start point and end point. In this case, the presentation position of the fixation target or the imaging optical system may be displaced in one predetermined direction.

In a case where the position adjustment unit is driven and a plurality of the front images are captured by being triggered when a trigger signal is input, the synthetic front image may be updated and displayed. For example, every time the front image is newly acquired after the trigger signal is input, the synthetic front image with one or more front images captured so far may be generated by the image processing unit, and may be displayed on a monitor.

Each of the front images may be automatically captured when the optical axis of the imaging optical system and the visual axis of the subject's eye are in a predetermined position relationship. Only in a case of the predetermined position relationship, the control unit may receive the release operation performed by the examiner so that the front image can be manually captured. In each case, the position relationship for capturing each front image may be designated by the examiner, or may be constant for each synthetic front image. For example, in a case where the position relationship is designated by the examiner, a step of designating and registering a target position of the objective lens reflected image in each front image used for the synthesis on a certain front image (for example, on the observation image) may be performed before the front image is captured or after some of the front images are captured. For example, whether or not the optical axis of the imaging optical system and the visual axis of the subject's eye are in the predetermined position relationship may be detected based on the control amount of the position adjustment unit or the front image obtained as the observation image.

However, in acquiring each front image including the first front image or the second front image, the position relationship between the optical axis of the imaging optical system and the visual axis of the subject's eye does not need to be associated with each front image in advance. For example, while the position relationship between the optical axis of the imaging optical system and the visual axis of the subject's eye is adjusted, the control unit may capture a plurality of the front images whose positions of the objective lens reflected images are different from each other with respect to the tissue of the subject's eye in the image, and may decide afterwards the front images to be used for the synthesis (including the first front image and the second front image) from among a plurality of the captured front images. A method of determining afterwards will be described later.

### <Guide Information Displayed on Monitor>

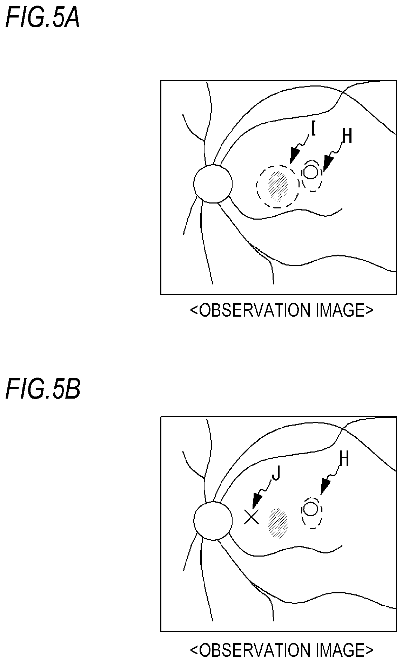

The guide information may be graphical information displayed on a monitor. For example, as illustrated in Fig. 5, the guide information may be information superimposed on the observation image. For example, the guide information may be information (for example, a graphic H) indicating a position of the objective lens reflected image in previously captured images in the first front image and the second front image. The graphic H is displaced on the observation image in accordance with the position relationship between the optical axis of the imaging optical system and the visual axis of the subject's eye. On the other hand, the actually reflected object image is displayed at a prescribed position on the observation image. Accordingly, the imaging is performed by adjusting the position relationship so that the objective lens reflected image and the graphic H in the observation image are in a separated state. In this manner, it is possible to obtain a set of the front images suitable for the synthetic front image. In this case, a graphic I together with the graphic H may be displayed as the guide information in a region surrounding the objective lens reflected image in the observation image. For example, the graphic I is information indicating a region where the graphic H should not be disposed, and it is preferable to capture the image at a position avoided from this region. For example, the control unit may automatically capture the front image by being triggered when the graphic H is no longer superimposed on the objective lens reflected image on the observation image or on the graphic I.

Information (for example, a graphic J) indicating a target position of the graphic H for obtaining the front image supposed to be captured may be displayed together with the graphic H as the guide information. The graphic J is displayed at a prescribed position on the observation image, regardless of the position relationship between the optical axis of the imaging optical system and the visual axis of the subject's eye. The image is captured by adjusting the position relationship so that the graphic H is superimposed on the graphic J. In this manner, a set of the front images suitable for the synthetic front image may be obtained. In this case, the control unit may automatically capture the front image by being triggered when the graphic H is completely superimposed on the graphic J.

The guide information may employ a display mode using a color switching method for indicating whether or not the position of the previously captured objective lens reflected image in the first front image and the second front image is separated from the objective lens reflected image on the observation image. For example, colors of the graphic displayed concurrently with the observation image may be switched therebetween.

### <Selection of Image used for Synthesis>

As described above, the first front image and the second front image, which are used to obtain the synthetic front image, may be obtained as a control result of the above-described imaging operation. However, the present disclosure is not necessarily limited thereto. For example, the control unit may select the first front image and the second front image afterwards from a plurality of the previously acquired front images whose positions of the objective lens reflected images are different from each other with respect to the subject's eye. In this case, the control unit may select two front images in which the objective lens reflected image in each front image is located in the superimposed portion of the front images and which are in a relationship where the objective lens reflected images are not superimposed on each other, as the first front image and the second front image, respectively.

### <Restraining Defective Reflected Image caused by Incident Angle of Illumination Light and Fundus Shape>

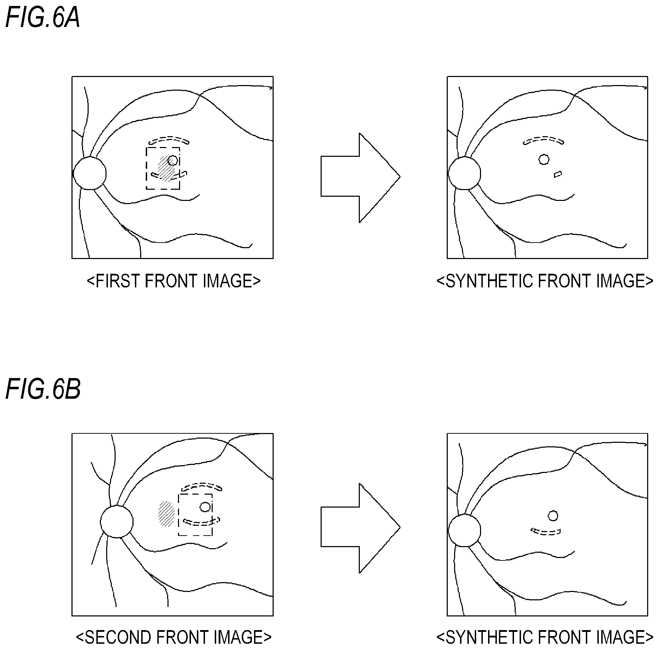

In a case where the front image is the fundus front image, the reflected image caused by an incident angle of the illumination light and a fundus shape (hereinafter referred to as a "second reflected image") is reflected as an artifact on the fundus front image in some cases. For example, an example of the second reflected image includes macular ring reflex illustrated in Fig. 6. The macular ring reflex is the reflected image surrounding the macula, which is caused by a recess in the retina in the vicinity of the macula.

Two images having different position relationships between the optical axis of the imaging optical system and the visual axis of the subject's eye have different incident angles of the illumination light with respect to each position in the fundus. Therefore, the second reflected image appearing in one of the first front image and the second front image does not appear on the other image in some cases. In this case, it is conceivable that a portion of the second reflected image appearing in one image is superimposed on the objective lens reflected image (Condition 1), and that the portion of the second reflected image is located in the boundary of the region to be replaced with the other image (or replaced with the other region) (Condition 2). In this case, a case is conceivable where the second reflected image in the synthetic front image is expressed as a partially defective image compared to the second reflected image in the original front image.

If the partially defective second reflected image is present, there is a possibility that an examiner may misunderstand the second reflected image as the tissue of the fundus rather than the artifact. In some cases, the second reflected image is used as an indicator of the fundus shape and the progress of aging when image diagnosis is performed using the fundus front image. Therefore, in the synthetic front image, it is more preferable that the second reflected image is perfect without any defect or the second reflected image is not reflected thereon.

In contrast, the ophthalmic imaging device may have a configuration for forming the synthetic front image which does not include the second reflected image, or the synthetic front image which includes the second reflected image having no defect compared to the original front image.

For example, the ophthalmic imaging device may have a detection unit which detects the second reflected image from the front image. The control unit may adjust the position relationship when at least one of the first front image and the second front image is acquired based on the detection result of the second reflected image detected by the detection unit, or may output the guide information. More specifically, in a case where a portion of the second reflected image detected by the detection unit satisfies a "defect condition" in the synthetic front image, the control unit may adjust the position relationship, or may output the guide information. For example, the "defect condition" may be defined as the above-described (Condition 1) or (Condition 2).

For example, the control unit may have a configuration in which the objective lens reflected image or the fundus front image which does not include the second reflected image in the boundary of the image region to be replaced with the region including the objective lens reflected image is set and acquired as the first front image and the second front image. For example, in a case where it is determined that the "defect condition" is satisfied as a result of detection by the detection unit from the observation image, the control unit performs the control for further displacing the position relationship between the optical axis of the imaging optical system and the visual axis of the subject's eye, or outputs the guide information. In this manner, the first front image or the second front image may be captured using the position relationship where the observation image does not satisfy the "defect condition". In a case where it is determined that the "defect condition" is satisfied after the detection unit detects the defective image in the first front image and the second front image which are captured once, the front image is captured again by changing the position relationship from the position relationship used for the previous imaging. Whether or not the second reflected image is reflected on the front image is affected by the wavelength of the illumination light, the output of the light source, and the gain. Thus, even if the second reflected image does not appear in the observation image, the second reflected image appears in the captured image in some cases. In this case, it is useful to capture the front image again.

### <Application to Image Processing Program>

The present disclosure may be applied to an image processing program. In this case, for example, the image processing program is executed by an ophthalmic imaging device having the above-described imaging optical system or a processor of any computer among general-purpose computers such as a computer (for example, a server computer), a PC, a tablet, or a smartphone connected to the ophthalmic imaging device via a network. The image processing program is stored in a memory of any one of the above-described computers. In either case, the front image captured by the ophthalmic imaging device having the imaging optical system is processed.

The image processing program is executed by the processor, thereby performing at least an image processing step. In the image processing step, the synthetic front image is obtained by synthesizing at least two of the first front image and the second front image from the front images stored in the memory. In the memory, the front images captured by the ophthalmic imaging device are stored in advance or by the imaging. The content of the synthesizing process is the same as that according to the embodiment in the ophthalmic imaging device, and thus, detailed description thereof will be omitted.

Next, according to the invention, a device will be described in which the ophthalmic imaging device according to the present disclosure is applied to a scanning laser ophthalmoscope (SLO). A scanning laser ophthalmoscope (hereinafter, referred to as "SLO") 1 scans the fundus with the illumination light (laser light) and receives returning light of the illumination light from the fundus. In this manner, the scanning laser ophthalmoscope acquires the front image of the fundus. SLO 1 may be a device integrated with other ophthalmic devices such as an optical coherence tomography (OCT) device and a perimeter.

SLO 1 acquires the front image of the fundus by scanning the fundus with the illumination light (laser light) and receiving fundus reflected light. SLO 1 two-dimensionally scans the fundus with the laser light focused in a spot shape on an observation surface, based on an operation of the scanning unit. However, the present disclosure is not necessarily limited thereto. For example, the present disclosure may be applied to a so-called line scan SLO. In this case, based on the operation of the scanning unit, the observation surface is one-dimensionally scanned with a linear laser luminous flux.

### <External Configuration of Device>

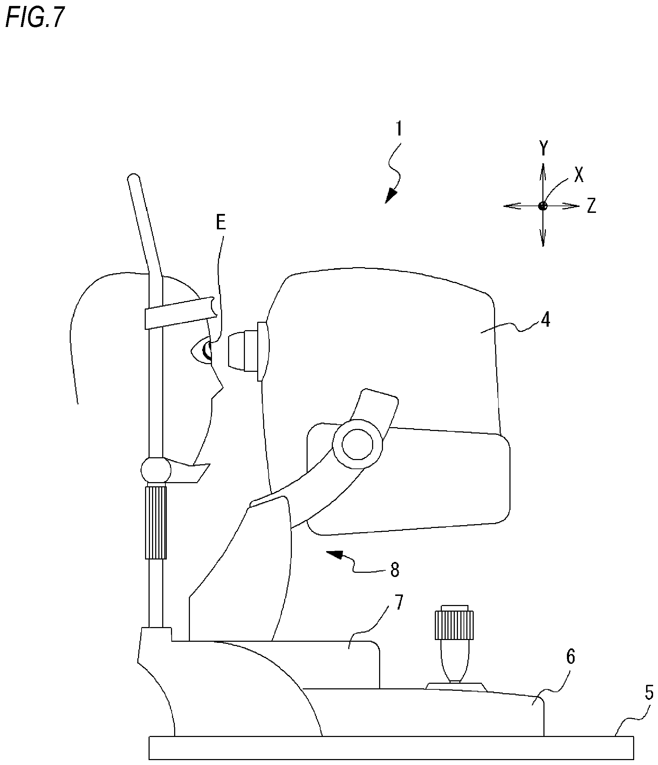

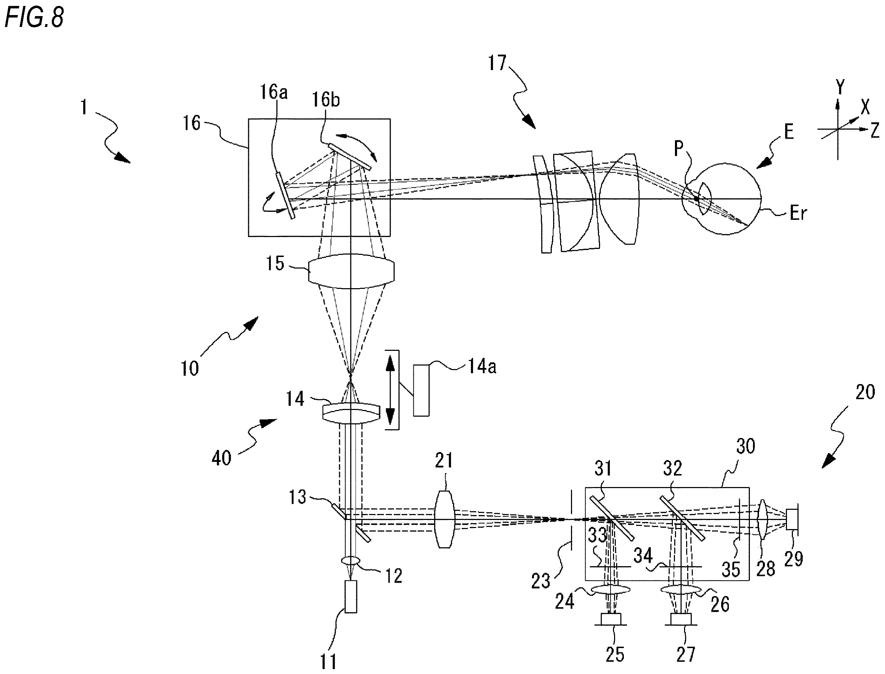

First, referring to Figs. 7 to 9, a schematic configuration of SLO 1 will be described. As illustrated in Fig. 7, SLO 1 mainly includes an imaging unit 4. The imaging unit 4 includes at least a major optical system in SLO 1 (refer to Fig. 8).

SLO 1 may include at least any one of an alignment mechanism and an imaging optical system drive mechanism. For example, the alignment mechanism in SLO 1 is used in order to locate a position where a pivot point of the laser light is formed to a position suitable to a subject's eye E. The alignment mechanism adjusts a relative position between the subject's eye and the imaging unit 4 (imaging optical system). The alignment mechanism may adjust the position relationship between the subject's eye and the imaging unit 4 (imaging optical system) in each direction of X (lateral), Y (vertical), and Z (longitudinal) directions. In a specific example illustrated in Fig. 7, a base 5, a movable carriage 6, and a Z-drive mechanism 7 are used as the alignment mechanism in the horizontal direction (XZ-direction) and the vertical direction (Y-direction). That is, the movable carriage 6 is movable on the base 5 in the XZ-direction in a state where the imaging unit 4 is placed thereon. The Z-drive mechanism 7 is placed on the movable carriage 6, and displaces the imaging unit 4 in the Z-direction. In this manner, the position relationship in the X, Y and Z directions between the subject's eye and the imaging unit 4 (imaging optical system) is adjusted.

The imaging optical system drive mechanism is a mechanical drive mechanism for adjusting an imaging position (imaging position on the subject's eye) of the front image acquired by the imaging unit 4. The imaging optical system drive mechanism may directly or indirectly support (for example, may hold) the imaging unit 4. In the overall ophthalmic imaging device, the imaging optical system drive mechanism changes the position relationship between the visual axis of the subject's eye E and an optical axis (also referred to as an imaging optical axis) of the imaging unit 4 (imaging optical system). In a case where the imaging optical system drive mechanism is applied to the fundus imaging device such as SLO 1, the imaging optical system drive mechanism adjusts an angle between an orientation of the visual axis of the subject's eye E and an orientation of the optical axis of the imaging unit 4. In this manner, the imaging position on the fundus is changed. As a specific example, in the specific example illustrated in Fig. 7, a swinging/tilting unit 8 is used as the imaging optical system drive mechanism. The swinging/tilting unit 8 causes the imaging unit 4 to laterally pivot around the subject's eye E. The swinging/tilting unit 8 causes the imaging unit 4 to rise to the subject's eye E.

One or both of the alignment mechanism and the imaging optical system drive mechanism may have an actuator which performs a predetermined operation based on a control signal so that the above-described operation is realized by controlling the drive of the actuator.

### <Imaging Optical System>

Next, the optical system of SLO 1 will be described. As illustrated in Fig. 8, SLO 1 has an irradiation optical system 10 and a light receiving optical system 20 (collectively, referred to as an "imaging optical system"). In the present embodiment, these optical systems 10 and 20 are disposed in the imaging unit 4. SLO 1 uses these optical systems 10 and 20 so as to capture the front image of the fundus. As described above, the imaging optical system of the SLO 1 may be the optical system using a point scan method, or may be the optical system using line scan method.

The irradiation optical system 10 includes at least a scanning unit (also referred to as an optical scanner) 16 and an objective lens system 17. As illustrated in Fig. 8, the irradiation optical system 10 further includes a laser light emitter 11, a collimating lens 12, a perforated mirror 13, a lens 14 (in the present embodiment, a portion of a diopter adjuster 40), and a lens 15. In a case of the point scan method, the optical scanner may be a two-dimensional optical scanner. The two-dimensional optical scanner two-dimensionally scans the fundus with the illumination light (detailed configuration will be described later). In a case of the line scan method, the optical scanner may be a one-dimensional optical scanner.

The laser light emitter 11 is a light source of the irradiation optical system 10. For example, the laser light emitter 11 may include at least one of a laser diode (LD) and a super luminescent diode (SLD). Although description of a detailed structure will be omitted, the laser light emitter 11 emits light having at least one wavelength range. In the present embodiment, the light having a plurality of colors is simultaneously or selectively emitted from the laser light emitter 11. For example, in the present embodiment, the light having four colors in total such as three colors of blue, green and red in a visible range and one color in an infrared region is emitted from the laser light emitter 11. For example, the three colors of blue, green and red in the visible range are used for color imaging. For example, the color imaging is performed by substantially simultaneously emitting the light having the three colors of blue, green, and red from the light source 11. Any one color out of the three colors in the visible range may be used for visible fluorescence imaging. For example, blue light may be used for fluorescein angiography (FAG) imaging which is one type of the visible fluorescence imaging. For example, light in the infrared region may be used for infrared fluorescence imaging in addition to infrared imaging using the fundus reflected light in the infrared region. For example, as the infrared fluorescence imaging, indocyanine green angiography (ICG) imaging is known. In this case, it is preferable that the infrared light emitted from the laser light source 11 is set to have a wavelength range different from the fluorescent wavelength of the indocyanine green angiography imaging.

The laser light is guided to a fundus Er by a light ray path illustrated in Fig. 8. That is, the laser light from the laser light emitter 11 passes through an opening formed in the perforated mirror 13 through the collimating lens 12. After passing through the lens 14 and the lens 15, the laser light travels toward the scanning unit 16. The laser light reflected by the scanning unit 16 passes through the objective lens system 17 and thereafter is used in irradiating the fundus Er of the subject's eye E. As a result, the laser light is reflected and scattered on the fundus Er. Alternatively, the laser light excites a fluorescent material present in the fundus, and causes fluorescence to be detected from the fundus. These types of light (that is, reflected and scattered light and fluorescence) are emitted from a pupil as the returning light.

In the present embodiment, the lens 14 illustrated in Fig. 8 is a portion of the diopter adjuster 40. The diopter adjuster 40 is used in order to correct (reduce) a diopter error of the subject's eye E. For example, the lens 14 can be moved by a drive mechanism 14a in a direction of the optical axis of the irradiation optical system 10. Depending on the position of the lens 14, each diopter of the irradiation optical system 10 and the light receiving optical system 20 is changed. Therefore, the position of the lens 14 is adjusted, thereby reducing the diopter error of the subject's eye E. As a result, a light focusing position of the laser light can be set to an observation site (for example, a retinal surface) of the fundus Er. For example, the diopter adjuster 40 may employ an optical system different from that in Fig. 8, such as a Badares optical system.

The scanning unit 16 is used in order to scan the fundus with the laser light emitted from the light source (laser light emitter 11). In the following description, unless otherwise specifically described, the scanning unit 16 includes two optical scanners which have mutually different scanning directions of the laser light. The two optical scanners are disposed at mutually different positions. That is, the scanning unit 16 includes an optical scanner 16a for main scanning (for example, for scanning in the X-direction) and an optical scanner 16b for sub-scanning (for scanning in the Y-direction). The optical scanner 16a for main scanning and the optical scanner 16b for sub-scanning may be respectively a resonant scanner and a galvano mirror. However, the present disclosure is not necessarily limited thereto, and in addition to other reflection mirrors (a galvano mirror, a polygon mirror, a resonant scanner or MEMS), an acousto optical modulator (AOM) which changes a light traveling (deflection) direction may be applied to the respective optical scanners 16a and 16b. The scanning unit 16 does not necessarily include a plurality of the optical scanners, and may include one optical scanner. As a single optical scanner which two-dimensionally scans the fundus with the laser light, it is proposed to utilize any one of the MEMS device and the acousto optical modulator (AOM), for example.

The objective lens system 17 serves as an objective optical system of SLO 1. The objective lens system 17 includes at least one lens. As illustrated in Fig. 8, the objective lens system 17 may be configured so that a plurality of lenses are combined with each other. The objective lens system 17 does not necessarily need to be the optical system including only a lens (in other words, a refractive element), and may include a mirror (in other words, a reflective element). The objective lens system 17 is used in order to guide the laser light used in scanning by the scanning unit 16 to the fundus Er. To that end, the objective lens system 17 forms a pivot point P around which the laser light passing through the scanning unit 16 pivots. The pivot point P is formed on an optical axis L1 of the irradiation optical system 10 at a position optically conjugate with the scanning unit 16 with regard to the objective lens system 17. In the present disclosure, "conjugation" is not necessarily limited to a complete conjugate relationship, and includes "substantial conjugation". That is, even in a case where both of these are disposed by being deviated from a complete conjugate position within an allowable range in relation to a use purpose (for example, observation or analysis) of the fundus image, the meaning is included in the "conjugation" in the present disclosure.

The laser light passes through the objective lens system 17 after passing through the scanning unit 16, thereby causing the fundus Er to be irradiated with the laser light via the pivot point P. Therefore, the laser light passing through the objective lens system 17 is caused to pivot around the pivot point P in response to the operation of the scanning unit 16. As a result, in the present embodiment, the fundus Er is two-dimensionally scanned with the laser light. The laser light used in irradiating the fundus Er is reflected at the light focusing position (for example, the retinal surface). In addition, the laser light is scattered by the tissue present in the front and rear of the light focusing position. The reflected light and the scattered light are respectively emitted from a pupil as parallel light.

Next, the light receiving optical system 20 will be described. The light receiving optical system 20 shares the objective lens system 17 with the irradiation optical system 10. The light receiving optical system 20 causes the light receiving element to receive the reflected light of the illumination light reflected on the subject's eye E via the objective lens system 17. As in this example, in SLO using the point scan method, the light receiving element may be a point sensor, for example. In a case of the line scan method, the light receiving element may be a line sensor, for example. The light receiving optical system 20 has one or more light receiving elements. For example, as illustrated in Fig. 8, the light receiving optical system 20 may have three light receiving elements 25, 27, and 29.

As illustrated in Fig. 8, the light receiving optical system 20 according to the present embodiment may share each member disposed from the objective lens system 17 to the perforated mirror 13 with the irradiation optical system 10. In this case, the light from the fundus is guided to the perforated mirror (in the present embodiment, an optical path diverging member) 13 through an optical path of the irradiation optical system 10. The perforated mirror 13 diverges the irradiation optical system 10 and the light receiving optical system 20 from each other. In the present embodiment, the light from the fundus Er is reflected on a reflective surface of the perforated mirror 13 so as to be guided to an independent optical path (optical path on the light receiving elements 25, 27, and 29 side) of the light receiving optical system 20. The light traveling toward the perforated mirror from an optical path common to the irradiation optical system 10 and the light receiving optical system 20 is likely to include noise light in a region in the vicinity of an optical axis L2 in the principal ray of the laser light. The noise light described herein mainly indicates the light from those other than imaging and observation targets, such as the light focusing position (for example, the retinal surface) of the fundus Er. Specifically, the noise light includes the reflected light from a cornea and the reflected light from the optical system inside the device. The perforated mirror 13 guides the light from the fundus Er to the independent optical path of the light receiving optical system 20 while removing at least a portion of the noise light. In the present embodiment, the perforated mirror 13 is disposed at a position conjugate with an anterior ocular segment. Therefore, the reflected light from the cornea and the reflected light from the optical system inside the device are removed by an opening of the perforated mirror 13. On the other hand, within the light from the fundus Er, the light passing through the periphery of the pupil is reflected on the reflective surface of the perforated mirror 13, and is guided to the independent optical path.

The optical path diverging member which diverges the irradiation optical system 10 and the light receiving optical system 20 from each other is not limited to the perforated mirror 13. For example, instead of the perforated mirror 13, a member replacing the opening and reflective surface with each other in the perforated mirror 13 can be used as an optical path diverging unit. However, for example, in a case where the member is applied to Fig. 8, it is further necessary to dispose the independent optical path (from the light source 11 to the perforated mirror 13) of the irradiation optical system 10 and the independent optical path (from the perforated mirror 13 to the light receiving elements 25, 27, and 29) of the light receiving optical system 20 by replacing both of these with each other. As the optical path diverging member, other members such as a beam splitter may be used.

The light receiving optical system 20 has a lens 21, a light blocking unit 22, a pinhole plate 23, and a light separator (light separating unit) 30 in a reflected light path of the perforated mirror 13. In addition, lenses 24, 26, and 28 are disposed between the light separator 30 and each of the light receiving elements 25, 27, and 29.

Although will be described in detail later, the light blocking unit 22 blocks the light in the vicinity of the optical axis L2 of the light receiving optical system 20. The light from a fundus conjugate plane passes through the light blocking unit 22, and at least a portion of the noise light is blocked by the light blocking unit 22.

In addition, the pinhole plate 23 is disposed on the fundus conjugate plane, and functions as a confocal diaphragm in SLO 1. That is, in a case where the diopter is properly corrected by the diopter adjuster 40, the light from the fundus Er after passing through the lens 21 is focused on the opening of the pinhole plate 23. The light from positions other than a focal point (or a focal plane) of the fundus Er is removed by the pinhole plate 23, and the remaining light (light from the focal point) is mainly guided to the light receiving elements 25, 27, and 29.

The light separator 30 separates the light from the fundus Er. In the present embodiment, the light from the fundus Er is separated by the light separator 30 in a wavelength-selective manner. In addition, the light separator 30 may also serve as an optical diverging unit which diverges the optical path of the light receiving optical system 20. For example, as illustrated in Fig. 8, the light separator 30 may include two dichroic mirrors (dichroic filters) 31 and 32 having different light separation characteristics (wavelength separation characteristics). The optical path of the light receiving optical system 20 is diverged into three by the two dichroic mirrors 31 and 32. In addition, each one of the light receiving elements 25, 27, and 29 is disposed in front of each diverged optical path.

For example, the light separator 30 separates the wavelength of the light from the fundus Er, and causes the three light receiving elements 25, 27, and 29 to receive the light having mutually different wavelength ranges. For example, the light having three colors of blue, green, and red may be received one by one by the light receiving elements 25, 27, and 29. In this case, a color image can be easily obtained based on the result of the light received by the light receiving elements 25, 27, and 29.

In addition, the light separator 30 may cause at least one of the light receiving elements 25, 27, and 29 to receive the light in the infrared region used in infrared imaging. In this case, for example, fluorescence used in fluorescence imaging and the light in the infrared region used in the infrared imaging may be received by mutually different light receiving elements.

The wavelength bands where the light receiving elements 25, 27, and 29 are sensitive may be different from each other. In addition, at least two of the light receiving elements 25, 27, and 29 may be sensitive to a common wavelength range. Each of the light receiving elements 25, 27, and 29 outputs a signal corresponding to the intensity of the received light (hereinafter, referred to as a received light signal). In the present embodiment, the received light signal is separately processed by each light receiving element so as to generate an image. That is, in the present embodiment, maximum three types of the fundus image are concurrently generated.

### <Fixation Optical System>

SLO 1 may also have a fixation optical system. In the example of Fig. 8, the fixation optical system is also used as the irradiation optical system 10. Visible light which is intermittently turned on from the laser light emitter 11 is used as a fixation light flux (in other words, a fixation target). Details will be described later.

### <Configuration of Control System>

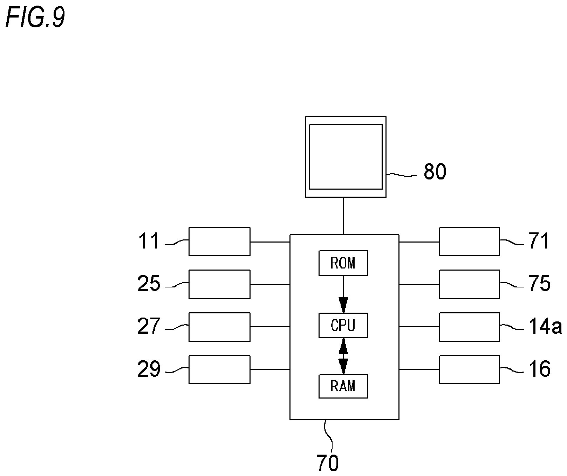

Next, referring to Fig. 9, a control system of SLO 1 will be described. In SLO 1, each unit is controlled by a control unit 70. The control unit 70 is a processing device (processor) having an electronic circuit to perform control processing and calculation processing of each unit of SLO 1. The control unit 70 is realized by a central processing unit (CPU) and a memory. The control unit 70 is electrically connected to a storage unit 71 via a bus. In addition, the control unit 70 is electrically connected to each unit such as the laser light emitter 11, the light receiving elements 25, 27, and 29, the drive mechanism 14a, the scanning unit 16, an input interface 75, and a monitor 80.

Various control programs and fixed data are stored in the storage unit 71. In addition, data may be temporarily stored in the storage unit 71. The image captured by SLO 1 may be stored in the storage unit 71. However, the present disclosure is not limited thereto. The captured image may be stored in an external storage device (for example, a storage device connected to the control unit 70 via LAN and WAN).

For the sake of convenience, in the present embodiment, the control unit 70 also serves as an image acquisition unit and an image processing unit. For example, based on the received signal output from any one of the light receiving elements 25, 27, and 29, the control unit 70 forms the fundus image. In addition, the image processing (image processing and analysis) for the fundus image is also performed by the control unit 70. As illustrated in Fig. 9, based on the signal from the respective light receiving elements 25, 27, and 29, the control unit 70 substantially simultaneously generates maximum three types of image. As a matter of course, the present disclosure is not limited to this example, and the image processing unit or the image acquisition unit may be a device separate from the control unit 70.

The control unit 70 acquires the fundus front image based on the fundus reflected light in the infrared region as the observation image, and displays the fundus front image on the monitor 80. The observation image is a moving image acquired on a substantially real time basis.

The control unit 70 controls the above-described respective members, based on an operation signal output from the input interface 75 (operation input unit). The input interface 75 is an operation input unit for receiving an operation of an examiner. For example, the input interface 75 may be a mouse and a keyboard.

### <Description of Operation>

In general, if a light source side lens surface of the lens which greatly bends the laser light is disposed close to a fundus conjugate plane (also referred to as an intermediate image plane) relating to the objective lens system, noise light which cannot be removed by the confocal diaphragm and the perforated mirror is likely to be generated. In some cases, the diopter is corrected in order to properly focus the laser light on the fundus. In this case, the fundus conjugate plane formed via the objective lens system moves in accordance with the correction amount of the diopter. For example, as the myopia of the subject's eye is severe, as the result of diopter correction, the fundus conjugate plane of the objective lens system moves closer to the subject's eye E (and the lens surface). In this way, it is also conceivable that the noise light is likely to be generated due to the influence of the diopter correction.

A harmful light removed portion (for example, a confocal diaphragm or a black spot board) disposed in the optical system reduces possibilities that the reflected image of the illumination light which is caused by the objective lens system may be reflected on the fundus image. However, a case is conceivable where a restraining effect of reflection and the light amount of the fundus reflected light guided to the light receiving element are in a trade-off relationship. Accordingly, it is conceivable to provide a design solution and an imaging condition which allow the reflection of the reflected image (hereinafter, for the sake of convenience, referred to as an "objective lens reflected image") by the objective lens system 17.

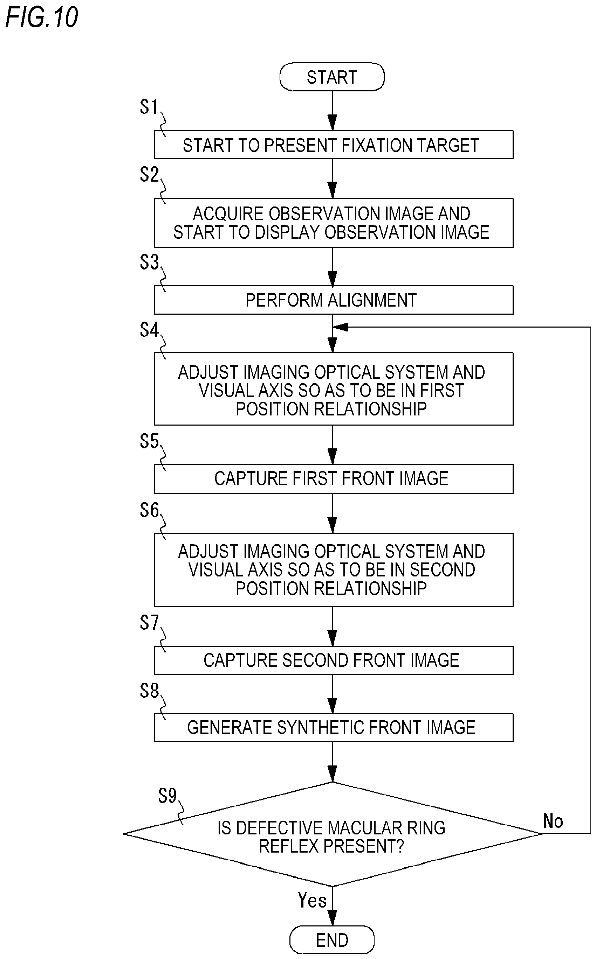

SLO 1 of this example employs image processing so as to reduce the reflection of the objective lens reflected image. A specific operation in SLO 1 will be described with reference to a flowchart illustrated in Fig. 10.

First, the control unit 70 starts to present the fixation target (S1), and starts to acquire the observation image (S2). Next, the alignment mechanism is automatically or manually driven based on the observation image so as to align the subject's eye with the device (S3).

After the alignment is completed, an imaging sequence (S4 to S7) is performed. In this imaging sequence, two front images of the first front image and the second front image are captured. In this example, the synthetic front image which does not include macular ring reflex as at least the second reflected image is generated.

In the imaging sequence of this example, when each front image is captured, the position relationship between the optical axis of the imaging optical system and the visual axis of the subject's eye is adjusted based on the observation image. First, the control unit 70 adjusts the position relationship between the optical axis of the imaging optical system and the visual axis of the subject's eye to a first position relationship (S4). Next, the first front image is captured (S5).

This example shows a case where a color fundus image can be obtained as the first front image and the second front image. However, the present disclosure is not limited to the color fundus image. The process of this example may be applied to other front images formed by the fundus reflected light, such as the infrared fundus image and the reflected image using visible light. For example, the control unit 70 stops irradiating the fundus with the infrared light emitted from the laser light emitter 11, and emits the visible lights having three colors of red, green, and blue as the illumination light for imaging at the same time (substantially at the same time). Based on the signal output from the three light receiving elements 25, 27 and 29, the fundus front image is formed so as to have each component of red, green and blue. These components are synthesized with each other, thereby forming the color fundus image.

Next, the control unit 70 adjusts the position relationship between the optical axis of the imaging optical system and the visual axis of the subject's eye to a second position relationship (S6). Next, the second front image is captured (S7).

In this example, the first position relationship and the second position relationship are adjusted by switching presentation positions of a fixation lamp. In this example, the visible light emitted from the laser light emitter 11 is used as the fixation target. Specifically, the control unit 70 intermittently emits the visible light from the laser light emitter 11 at timing corresponding to the presentation positions. The timing at which the visible light is emitted is changed while the scanning unit 16 scans one frame of the fundus image, thereby changing the presentation positions of the fixation target.

In this example, when the position relationship is adjusted to the first position relationship and the second position relationship, the macular ring reflex in the observation image may be detected by the control unit 70. In this example, the control unit 70 captures the first front image or the second front image by using a position relationship where no macular ring reflex is detected in the observation image. In a case where the macular ring reflex is detected in the observation image, the position relationship between the optical axis of the imaging optical system and the visual axis of the subject's eye is further adjusted.

After the first front image and the second front image are captured, the control unit 70 synthesizes the two front images, and obtains the synthetic front image (S8). In this example, the control unit 70 detects whether or not defective macular ring reflex is included in the synthetic front image. In a case where the defective macular ring reflex is included (S9: Yes), the control unit 70 changes the position relationship between the optical axis of the imaging optical system and the visual axis of the subject's eye so as to be different from the previous position relationship. In this manner, the control unit 70 may capture the first front image and the second front image again so as to form a new synthetic front image. In this example, through trial and error in the process of S4 to S9, the synthetic front image which does not include the defective macular ring reflex is formed. On the other hand, in a case where the control unit 70 determines that the synthetic front image does not include the macular ring reflex in the process of S8 (S9: No), the process may be completed.

Hitherto, the present disclosure has been described with reference to the embodiment. However, the content of the embodiment can be appropriately modified in embodying the present disclosure.

For example, the objective lens system in the ophthalmic imaging device may employ an interchangeable lens or a zoom lens, thereby adopting a configuration in which an imaging field angle is variable. In this case, in a case where the imaging field angle is set to a first imaging field angle, a clear objective lens reflected image is generated on the front image. At a second imaging field angle different from the first imaging field angle, a case is conceivable where a noticeable objective lens reflected image is not generated. Therefore, for example, a configuration may be adopted which includes the imaging sequence for generating the synthetic front image, based on a release operation, and which can switch between a first mode for performing a generation process of the synthetic front image and a second mode for capturing the fundus image by using the position relationship between the optical axis of the imaging optical system and the visual axis of the subject's eye, when the release operation is received, based on the release operation. The switching between the first mode and the second mode may be controlled based on a mode switching operation by an examiner. In addition, the modes may be automatically switched in accordance with lens exchange of the objective lens system or a zooming state of zooming. The interchangeable lens is not limited to the configuration in which the objective lens system is directly replaced with the other objective lens system as it is. A configuration may be adopted in which the imaging field angles are switched by attaching or detaching a lens attachment to or from the objective lens system.

In addition, and not forming part of the invention, the image processing for generating the above-described synthetic front image is not limitedly applied to SLO. In addition to SLO, the image processing may be applied to "an ophthalmic imaging device that includes an imaging optical system including the objective lens system including at least one lens, the irradiation optical system which irradiates the subject's eye with the illumination light emitted from the light source via the objective lens system, and the light receiving optical system including the light receiving element which receives the reflected light of the illumination light on the subject's eye via the objective lens system, and that generates the captured image based on the signal output from the light receiving element". This ophthalmic imaging device may be a fundus imaging device. In this case, the irradiation optical system irradiates the fundus of the subject's eye with the illumination light, and the light receiving optical system causes the light receiving element to receive the fundus reflected light of the illumination light. For example, as the fundus imaging device other than SLO, a fundus camera is conceivable. In addition, the ophthalmic imaging device may be an anterior ocular segment camera.

## Claims

1. An ophthalmic imaging device comprising:
an imaging optical system (4) that includes an irradiation optical system (10) configured to irradiate a subject's eye with illumination light emitted from a light source (11) via an objective lens system (17) including at least one lens and cause an optical scanner (16) to scan a tissue of the subject's eye with the illumination light, and a light receiving optical system (20) which has a light receiving element (29) for receiving reflected light of the illumination light reflected on the subject's eye via the objective lens system (17);
image acquisition means configured to generate and acquire a front image of the subject's eye based on a signal output from the light receiving element (29);
position adjustment means configured to adjust a position where a reflected image of the illumination light is formed by the objective lens system (17) for the tissue of the subject's eye in the front image by changing a position relationship between an optical axis of the imaging optical system and a visual axis of the subject's eye; and
image processing means configured to obtain a synthetic front image in which the reflected image is reduced compared to a first front image or a second front image, based on at least two images of the first front image and the second front image,
wherein a position of the reflected image with respect to the tissue of the subject's eye included in the first front image is different from that of the reflected image with respect to the tissue of the subject's eye included in the second front image,
wherein the second front image includes an area of the reflected area in the first front image,
wherein the image processing means configured to generate the synthetic front image by supplementing a region including the reflected image in the first front image, with an image region included in the second front image, which is an image region whose position with respect to the tissue of the subject's eye is the same as that of the region including the reflected image, **characterized in that**
the image processing means is configured to perform a process for smoothing a luminance change in a joining portion between the first front image and the second front image when the image processing means generates the synthetic front image, and
the ophthalmic imaging device is applied to a scanning laser ophthalmoscope.

2. The ophthalmic imaging device according to Claim 1, further comprising:
control means (70) configured to output guide information to guide an examiner or a subject to adjust the position relationship or to perform an operation for adjusting the position relationship by controlling the position adjustment means to acquire at least any one of the first front image and the second front image.

3. The ophthalmic imaging device according to Claim 2,
wherein the control means (70) is configured to perform an imaging sequence for acquiring the first front image and the second front image, and
wherein the first front image and the second front image are acquired based on each independent release operation.

4. The ophthalmic imaging device according to Claim 2,
wherein the control means (70) is configured to perform an imaging sequence for automatically and continuously acquire the first front image and the second front image.

5. The ophthalmic imaging device according to Claim 3 or 4,
wherein the control means (70) is configured to perform the imaging sequence so as to acquire one of the first front image and the second front image, and thereafter to automatically or manually acquire the other of the first front image and the second front image by automatically adjusting the position relationship.

6. The ophthalmic imaging device according to any one of Claims 2 to 5,
wherein the control means (70) is configured to adjust the position relationship or output the guide information to substantially capture the same imaging range in the first front image and the second front image.

7. The ophthalmic imaging device according to any one of Claims 2 to 6,
wherein the control means (70) is configured to adjust the position relationship or is configured to output the guide information so that positions where the reflected image is formed are not superimposed on each other in the first front image and the second front image.

8. The ophthalmic imaging device according to any one of Claims 2 to 7,
wherein as the guide information, the control means is configured to frequently acquire a graphic to guide the position relationship for at least one image supposed to be captured in the first front image and the second front image, based on a signal output from the light receiving element, and is configured to display the graphic so as to be superimposed on an observation image which is the front image to be displayed on a monitor (80) as a moving image.

9. The ophthalmic imaging device according to any one of Claims 2 to 8,
wherein the front image acquired by the image acquisition means is a fundus front image,
wherein the ophthalmic imaging device further has detection means configured to detect a second reflected image caused by a fundus shape and an incident angle of the illumination light, from the front image, and
wherein the control means (70) is configured to adjust the position relationship for acquiring at least any one of the first front image and the second front image, or is configured to output the guide information based on a detection result of the second reflected image.

10. The ophthalmic imaging device according to any one of Claims 1 to 9,
wherein the image processing means is configured to perform synthesis by adding at least the first front image and the second front image to each other.

11. The ophthalmic imaging device according to Claim 11,
wherein the image processing means is configured to perform the addition in such a way that the region including the reflected image in one or both of the first front image and the second front image is multiplied by a mask region for causing luminance distribution in the region to be flattened by background luminance.

12. The ophthalmic imaging device according to any one of Claims 1 to 9,
wherein the image processing means is configured to obtain the synthetic front image by setting the region including the reflected image in the first front image as a replacement target region and by replacing the replacement target region with an image region whose position with respect to the tissue of the subject eye's is the same as that of the replacement target region in the second front image.

13. The ophthalmic imaging device according to any one of Claims 1, 2 and 10 to 12, further comprising:
selection means configured to select the first front image and the second front image from the front images which are in a relationship where the regions of the reflected images are not superimposed on each other with respect to the tissue of the subject's eye, in a plurality of the front images which are acquired in advance and whose positions of the reflected images are different from each other with respect to the tissue of the subject's eye.

14. A method of generating an ophthalmic synthetic image using an ophthalmic imaging device configured to irradiate a subject's eye with illumination light emitted from a light source (11) via an objective lens system (17) including at least one lens so as to capture a front image of the subject's eye, based on reflected light of the illumination light reflected on the subject's eye via the objective lens system (17), the method comprising:
an imaging step of capturing, by the ophthalmic imaging device, a plurality of the front images whose positions of reflected images of the illumination light emitted by the objective lens system are different from each other with respect to a tissue of the subject's eye;
a front image acquisition step of storing the plurality of the front images captured in the imaging step in a memory of a computer; and
an image processing step of obtaining a synthetic front image based on at least two images of a first front image and a second front image acquired from the plurality of the front image stored in the memory,
wherein a position of the reflected image with respect to the tissue of the subject's eye included in the first front image is different from that of the reflected image with respect to the tissue of the subject's eye included in the second front image,
wherein the second front image includes an area of the reflected area in the first front image
wherein the image processing step generates the synthetic front image by supplementing a region including the reflected image in the first front image, with an image region included in the second front image, which is an image region whose position with respect to the tissue of the subject's eye is the same as that of the region including the reflected image, **characterized in that**
the image processing step performs a process for smoothing a luminance change in a joining portion between the first front image and the second front image when the image processing step generates the synthetic front image, and
the ophthalmic imaging device is applied to a scanning laser ophthalmoscope.

## Patentansprüche

1. Ophthalmische Abbildungsvorrichtung umfassend:
ein optisches Abbildungsoptiksystem (4), das ein optisches Bestrahlungssystem (10), das konfiguriert ist, um ein Auge eines Objekts mit Beleuchtungslicht zu bestrahlen, das von einer Lichtquelle (11) über ein objektives Linsensystem (17) ausgestrahlt ist, das mindestens eine Linse aufweist, und einen optischen Scanner (16) zum Scannen eines Gewebes des Objektauges mit dem Beleuchtungslicht zu bewirken, und ein optisches Lichtempfangssystem (20) aufweist, das ein Lichtempfangselement (29) zum Empfangen von reflektiertem Licht des Beleuchtungslichts aufweist, das über das objektive Linsensystem (17) auf das Objektauge reflektiert ist;
ein Bilderfassungsmittel, das konfiguriert ist, um ein Frontbild des Subjektauges basierend auf einem von dem Lichtempfangselement (29) ausgegebenen Signal zu generieren und zu empfangen;
Positionseinstellmittel, das konfiguriert ist, um eine Position einzustellen, an der ein reflektiertes Bild des Beleuchtungslichts durch das objektive Linsensystem (17) für das Gewebe des Subjektauges in dem Frontbild geformt ist, indem eine Positionsbeziehung zwischen einer optischen Achse des optischen Abbildungsoptiksystems und einer visuellen Achse des Objektauges geändert wird; und
Bildverarbeitungsmittel, das konfiguriert ist, um ein synthetisches Frontbild zu erhalten, in dem das reflektierte Bild im Vergleich zu einem ersten Frontbild oder einem zweiten Frontbild reduziert ist, basierend auf mindestens zwei Bildern des ersten Frontbildes und des zweiten Frontbildes,
wobei eine Position des reflektierten Bildes in Bezug auf das Gewebe des Objektauges, das in dem ersten Frontbild enthalten ist, unterschiedlich zu der des reflektierten Bildes in Bezug auf das Gewebe des Objektauges ist, das in dem zweiten Frontbild enthalten ist,
wobei das zweite Frontbild einen Bereich des reflektierten Bereichs in dem ersten Frontbild aufweist,
wobei das Bildverarbeitungsmittel konfiguriert ist, um das synthetische Frontbild zu erzeugen, indem ein Abschnitt, der das reflektierte Bild in dem ersten Frontbild aufweist, mit einem Bildabschnitt ergänzt wird, der in dem zweiten Frontbild enthalten ist, der ein Bildabschnitt ist, dessen Position in Bezug auf das Gewebe des Objektauges dieselbe ist wie die des Abschnittes, der das reflektierte Bild aufweist, **dadurch gekennzeichnet, dass**
das Bildverarbeitungsmittel konfiguriert ist, um einen Prozess zum Glätten einer Luminanzänderung in einem Verbindungsabschnitt zwischen dem ersten Frontbild und dem zweiten Frontbild durchzuführen, wenn das Bildverarbeitungsmittel das synthetische Frontbild generiert, und
die ophthalmische Abbildungsvorrichtung auf ein scannendes Laser-Ophthalmoskop verwendet wird.

2. Ophthalmische Abbildungsvorrichtung nach Anspruch 1, ferner umfassend:
ein Steuermittel (70), das zum Ausgeben von Führungsinformationen konfiguriert ist, sodass ein Prüfer oder ein Objekt dazu angeleitet wird, die Positionsbeziehung einzustellen oder eine Betätigung zum Einstellen der Positionsbeziehung durchzuführen, indem das Positionseinstellmittel zum Erfassen mindestens eines von dem ersten Frontbild und dem zweiten Frontbild gesteuert wird.

3. Ophthalmische Abbildungsvorrichtung nach Anspruch 2,
wobei das Steuermittel (70) konfiguriert ist, um eine Abbildungssequenz zum Erfassen des ersten Frontbildes und des zweiten Frontbildes durchzuführen, und
wobei das erste Frontbild und das zweite Frontbild basierend auf jeder unabhängigen Freigabenbetätigung erfasst werden.

4. Ophthalmische Abbildungsvorrichtung nach Anspruch 2,
wobei das Steuermittel (70) konfiguriert ist, um eine Abbildungssequenz zur automatischen und kontinuierlichen Erfassung des ersten Frontbildes und des zweiten Frontbildes durchzuführen.

5. Ophthalmische Abbildungsvorrichtung nach Anspruch 3 oder 4,
wobei das Steuermittel (70) zum Durchführen der Abbildungssequenz konfiguriert ist, sodass ein Bild von dem ersten Frontbild und dem zweiten Frontbild erfasst wird und anschließend das andere Bild von dem ersten Frontbild und dem zweiten Frontbild automatisch oder manuell erfasst wird, indem die Positionsbeziehung automatisch eingestellt wird.

6. Ophthalmische Abbildungsvorrichtung nach einem der Ansprüche 2 bis 5,
wobei das Steuermittel (70) zum Einstellen der Positionsbeziehung oder zum Ausgeben der Führungsinformationen konfiguriert ist, um im Wesentlichen denselben Abbildungsbereich in dem ersten Frontbild und dem zweiten Frontbild zu erfassen.

7. Ophthalmische Abbildungsvorrichtung nach einem der Ansprüche 2 bis 6,
wobei das Steuermittel (70) zum Einstellen der Positionsbeziehung konfiguriert ist oder zum Ausgeben der Führungsinformationen konfiguriert ist, sodass die Positionen, an denen das reflektierte Bild geformt ist, in dem ersten Frontbild und dem zweiten Frontbild nicht überlagert sind.

8. Ophthalmische Abbildungsvorrichtung nach einem der Ansprüche 2 bis 7,
wobei als die Führungsinformationen das Steuermittel konfiguriert ist, um regelmäßig eine Grafik zu erfassen, um die Positionsbeziehung für mindestens ein Bild, das in dem ersten Frontbild und dem zweiten Frontbild erfasst wird, basierend auf einem Signal, das von dem Lichtempfangselement ausgegeben wird, zu führen, und zum Anzeigen der Grafik konfiguriert ist, sodass sie einem Beobachtungsbild überlagert wird, das das Frontbild ist, das auf einem Monitor (80) als ein sich bewegendes Bild angezeigt wird.

9. Ophthalmische Abbildungsvorrichtung nach einem der Ansprüche 2 bis 8,
wobei das Frontbild, das von dem Bilderfassungsmittel erfasst ist, ein Fundus-Frontbild ist,
wobei die ophthalmische Abbildungsvorrichtung ferner ein Erfassungsmittel aufweist, das konfiguriert ist, um ein zweites reflektiertes Bild, das durch eine Fundusform und einen Einfallswinkel des Beleuchtungslichts verursacht wird, von dem Frontbild zu erfassen, und
wobei das Steuermittel (70) konfiguriert ist, um die Positionsbeziehung zum Erfassen mindestens eines von dem ersten Frontbild und dem zweiten Frontbild einzustellen, oder konfiguriert ist, um die Führungsinformationen basierend auf einem Erfassungsergebnis des zweiten reflektierten Bildes auszugeben.

10. Ophthalmische Abbildungsvorrichtung nach einem der Ansprüche 1 bis 9,
wobei das Bildverarbeitungsmittel konfiguriert ist, um eine Synthese durchzuführen, indem mindestens das erste Frontbild und das zweite Frontbild zueinander addiert werden.

11. Ophthalmische Abbildungsvorrichtung nach Anspruch 11,
wobei das Bildverarbeitungsmittel konfiguriert ist, um die Addition durchzuführen, indem der Abschnitt, der das reflektierte Bild in einem oder beiden von dem ersten Frontbild und dem zweiten Frontbild aufweist, mit einem Maskenabschnitt multipliziert wird, um zu bewirken, dass Luminanzverteilung in dem Abschnitt durch die Hintergrundluminanz abgeflacht wird.

12. Ophthalmische Abbildungsvorrichtung nach einem der Ansprüche 1 bis 9,
wobei das Bildverarbeitungsmittel konfiguriert ist, um das synthetische Frontbild zu erhalten, indem der Bereich, der das reflektierte Bild im ersten Frontbild aufweist, als Ersatz-Zielbereich eingestellt wird und indem der Ersatz-Zielbereich durch einen Bildbereich ersetzt wird, dessen Position in Bezug auf das Gewebe des Objektauges dieselbe wie die des Ersatz-Zielbereichs in dem zweiten Frontbild ist.

13. Ophthalmische Abbildungsvorrichtung nach einem der Ansprüche 1, 2 und 10 bis 12, ferner umfassend:
Auswahlmittel, das zum Auswählen des ersten Frontbildes und des zweiten Frontbildes aus den Frontbildern konfiguriert ist, die sich in einer Beziehung befinden, bei der die Abschnitte der reflektierten Bilder in Bezug auf das Gewebe des Objektauges nicht übereinander liegen, in einer Mehrzahl von Frontbildern, die im Voraus erfasst sind und deren Positionen der reflektierten Bilder sich in Bezug auf das Gewebe des Objektauges unterschiedlich voneinander sind.

14. Verfahren zum Erzeugen eines ophthalmischen synthetischen Bildes unter Verwendung einer ophthalmischen Abbildungsvorrichtung, die konfiguriert ist, um ein Auge eines Objekts mit Beleuchtungslicht zu bestrahlen, das von einer Lichtquelle (11) über ein objektives Linsensystem (17) ausgestrahlt wird, das mindestens eine Linse aufweist, sodass ein Frontbild des Objektauges basierend auf dem reflektierten Licht des Beleuchtungslichts, das über das objektive Linsensystem (17) auf das Objektauge reflektiert wird, erfasst wird, wobei das Verfahren aufweist:
einen Abbildungsschritt zum Erfassen, durch die ophthalmische Abbildungsvorrichtung, einer Mehrzahl von Frontbildern, deren Positionen von reflektierten Bildern des Beleuchtungslichts, das durch das objektive Linsensystem ausgestrahlt wird, in Bezug auf ein Gewebe des Subjektauges unterschiedlich sind;
einen Frontbild-Erfassungsschritt zum Speichern der Mehrzahl der Frontbilder, die bei dem Abbildungsschritt aufgenommen wurden, in einen Speicher eines Computers; und
einen Bildverarbeitungsschritt zum Erhalten eines synthetischen Frontbildes basierend auf mindestens zwei Bildern eines ersten Frontbildes und eines zweiten Frontbildes, die aus der Mehrzahl der in dem Speicher gespeicherten Frontbilder erfasst wurden,
wobei eine Position des reflektierten Bildes in Bezug auf das Gewebe des Objektauges, das in dem ersten Frontbild enthalten ist, unterschiedlich zu der des reflektierten Bildes in Bezug auf das Gewebe des Objektauges ist, das in dem zweiten Frontbild enthalten ist,
wobei das zweite Frontbild einen Bereich des reflektierten Bereichs in dem ersten Frontbild aufweist,
wobei der Bildverarbeitungsschritt das synthetische Frontbild generiert, indem ein Abschnitt, der das reflektierte Bild in dem ersten Frontbild aufweist, mit einem Bildabschnitt ergänzt wird, der in dem zweiten Frontbild enthalten ist, der ein Bildabschnitt ist, dessen Position in Bezug auf das Gewebe des Objektauges dieselbe ist wie die des Abschnittes, der das reflektierte Bild aufweist, **dadurch gekennzeichnet, dass**
der Bildverarbeitungsschritt einen Prozess zum Glätten einer Luminanzänderung in einem Verbindungsabschnitt zwischen dem ersten Frontbild und dem zweiten Frontbild durchführt, wenn der Bildverarbeitungsschritt das synthetische Frontbild generiert, und
die ophthalmische Abbildungsvorrichtung auf ein scannendes Laser-Ophthalmoskop verwendet wird.

## Revendications

1. Dispositif d'imagerie ophtalmique comprenant :
un système optique d'imagerie (4) qui comprend un système optique d'irradiation (10) configuré pour irradier l'oeil d'un sujet avec une lumière d'éclairage émise par une source lumineuse (11) par l'intermédiaire d'un système d'objectif (17) comprenant au moins une lentille et amener un lecteur optique (16) à balayer un tissu de l'oeil du sujet avec la lumière d'éclairage, et un système optique de réception de lumière (20) qui a un élément de réception de lumière (29) pour recevoir une lumière réfléchie de la lumière d'éclairage réfléchie sur l'oeil du sujet par l'intermédiaire du système d'objectif (17) ;
un moyen d'acquisition d'image configuré pour générer et acquérir une image frontale de l'oeil du sujet sur la base d'un signal émis par l'élément de réception de lumière (29) ;
un moyen d'ajustement de position configuré pour ajuster une position où une image réfléchie de la lumière d'éclairage est formée par le système d'objectif (17) pour le tissu de l'oeil du sujet dans l'image frontale en changeant une relation de position entre un axe optique du système optique d'imagerie et un axe visuel de l'oeil du sujet ; et
un moyen de traitement d'image configuré pour obtenir une image frontale synthétique dans laquelle l'image réfléchie est réduite par rapport à une première image frontale ou à une deuxième image frontale, sur la base d'au moins deux images de la première image frontale et de la deuxième image frontale,
dans lequel une position de l'image réfléchie par rapport au tissu de l'oeil du sujet inclus dans la première image frontale est différente de celle de l'image réfléchie par rapport au tissu de l'oeil du sujet inclus dans la deuxième image frontale,
dans lequel la deuxième image frontale comprend une zone de la zone réfléchie dans la première image frontale,
dans lequel le moyen de traitement d'image étant configuré pour générer l'image frontale synthétique en complétant une région comprenant l'image réfléchie dans la première image frontale, avec une région d'image incluse dans la deuxième image frontale, qui est une région d'image dont la position par rapport au tissu de l'oeil du sujet est la même que celle de la région comprenant l'image réfléchie, **caractérisé en ce que**
le moyen de traitement d'image est configuré pour exécuter un processus de lissage d'un changement de luminance dans une partie de jonction entre la première image frontale et la deuxième image frontale lorsque le moyen de traitement d'image génère l'image frontale synthétique, et
le dispositif d'imagerie ophtalmique est appliqué à un ophtalmoscope laser à balayage.

2. Dispositif d'imagerie ophtalmique selon la revendication 1, comprenant en outre :
un moyen de commande (70) configuré pour émettre des informations de guidage pour guider un examinateur ou un sujet pour ajuster la relation de position ou pour exécuter une opération d'ajustement de la relation de position en commandant le moyen d'ajustement de position afin d'acquérir au moins l'une quelconque de la première image frontale et de la deuxième image frontale.

3. Dispositif d'imagerie ophtalmique selon la revendication 2,
dans lequel le moyen de commande (70) est configuré pour exécuter une séquence d'imagerie afin d'acquérir la première image frontale et la deuxième image frontale, et
dans lequel la première image frontale et la deuxième image frontale sont acquises sur la base de chaque opération de libération indépendante.

4. Dispositif d'imagerie ophtalmique selon la revendication 2,
dans lequel le moyen de commande (70) est configuré pour exécuter une séquence d'imagerie pour acquérir automatiquement et en continu la première image frontale et la deuxième image frontale.

5. Dispositif d'imagerie ophtalmique selon la revendication 3 ou 4,
dans lequel le moyen de commande (70) est configuré pour exécuter la séquence d'imagerie de manière à acquérir l'une de la première image frontale et de la deuxième image frontale, et ensuite pour acquérir automatiquement ou manuellement l'autre de la première image frontale et de la deuxième image frontale en ajustant automatiquement la relation de position.

6. Dispositif d'imagerie ophtalmique selon l'une quelconque des revendications 2 à 5,
dans lequel le moyen de commande (70) est configuré pour ajuster la relation de position ou émettre les informations de guidage pour capturer sensiblement la même plage d'imagerie dans la première image frontale et la deuxième image frontale.

7. Dispositif d'imagerie ophtalmique selon l'une quelconque des revendications 2 à 6,
dans lequel le moyen de commande (70) est configuré pour ajuster la relation de position ou est configuré pour émettre les informations de guidage de sorte que les positions où l'image réfléchie est formée ne se superposent pas l'une sur l'autre dans la première image frontale et la deuxième image frontale.

8. Dispositif d'imagerie ophtalmique selon l'une quelconque des revendications 2 à 7,
dans lequel, en tant qu'informations de guidage, le moyen de commande est configuré pour acquérir fréquemment un graphique pour guider la relation de position pour au moins une image supposée être capturée dans la première image frontale et la deuxième image frontale, sur la base d'un signal émis par l'élément de réception de lumière, et est configuré pour afficher le graphique de manière à être superposé sur une image d'observation qui est l'image frontale à afficher sur un écran (80) en tant qu'image animée.

9. Dispositif d'imagerie ophtalmique selon l'une quelconque des revendications 2 à 8,
dans lequel l'image frontale acquise par le moyen d'acquisition d'image est une image frontale de fond d'oeil,
dans lequel le dispositif d'imagerie ophtalmique a en outre un moyen de détection configuré pour détecter une deuxième image réfléchie provoquée par une forme de fond d'oeil et un angle d'incidence de la lumière d'éclairage, à partir de l'image frontale, et
dans lequel le moyen de commande (70) est configuré pour ajuster la relation de position pour acquérir au moins l'une quelconque de la première image frontale et de la deuxième image frontale, ou est configuré pour émettre les informations de guidage sur la base d'un résultat de détection de la deuxième image réfléchie.

10. Dispositif d'imagerie ophtalmique selon l'une quelconque des revendications 1 à 9,
dans lequel le moyen de traitement d'image est configuré pour exécuter une synthèse en additionnant au moins la première image frontale et la deuxième image frontale l'une à l'autre.

11. Dispositif d'imagerie ophtalmique selon la revendication 11,
dans lequel le moyen de traitement d'image est configuré pour exécuter l'addition de telle manière que la région comprenant l'image réfléchie dans l'une et/ou l'autre de la première image frontale et de la deuxième image frontale est multipliée par une région de masque pour amener la distribution de luminance dans la région à être aplatie par la luminance de fond.

12. Dispositif d'imagerie ophtalmique selon l'une quelconque des revendications 1 à 9,
dans lequel le moyen de traitement d'image est configuré pour obtenir l'image frontale synthétique en définissant la région comprenant l'image réfléchie dans la première image frontale en tant que région cible de remplacement et en remplaçant la région cible de remplacement par une région d'image dont la position par rapport au tissu de l'oeil du sujet est la même que celle de la région cible de remplacement dans la deuxième image frontale.

13. Dispositif d'imagerie ophtalmique selon l'une quelconque des revendications 1, 2 et 10 à 12, comprenant en outre :
un moyen de sélection configuré pour sélectionner la première image frontale et la deuxième image frontale parmi les images frontales qui sont dans une relation où les régions des images réfléchies ne se superposent pas par rapport au tissu de l'oeil du sujet, dans une pluralité des images frontales qui sont acquises à l'avance et dont les positions des images réfléchies sont différentes les unes des autres par rapport au tissu de l'oeil du sujet.

14. Procédé de génération d'une image synthétique ophtalmique à l'aide d'un dispositif d'imagerie ophtalmique configuré pour irradier l'oeil d'un sujet avec une lumière d'éclairage émise par une source lumineuse (11) par l'intermédiaire d'un système d'objectif (17) comprenant au moins une lentille de manière à capturer une image frontale de l'oeil du sujet, sur la base d'une lumière réfléchie de la lumière d'éclairage réfléchie sur l'oeil du sujet par l'intermédiaire du système d'objectif (17), le procédé comprenant :
une étape d'imagerie consistant à capturer, par le dispositif d'imagerie ophtalmique, une pluralité des images frontales dont les positions d'images réfléchies de la lumière d'éclairage émise par le système d'objectif sont différentes les unes des autres par rapport à un tissu de l'oeil du sujet ;
une étape d'acquisition d'images frontales consistant à stocker la pluralité des images frontales capturées lors de l'étape d'imagerie dans une mémoire d'un ordinateur ; et
une étape de traitement d'image consistant à obtenir une image frontale synthétique sur la base d'au moins deux images d'une première image frontale et d'une deuxième image frontale acquises à partir de la pluralité d'images frontales stockées dans la mémoire,
dans lequel une position de l'image réfléchie par rapport au tissu de l'oeil du sujet inclus dans la première image frontale est différente de celle de l'image réfléchie par rapport au tissu de l'oeil du sujet inclus dans la deuxième image frontale,
dans lequel la deuxième image frontale comprend une zone de la zone réfléchie dans la première image frontale,
dans lequel l'étape de traitement d'image génère l'image frontale synthétique en complétant une région comprenant l'image réfléchie dans la première image frontale, avec une région d'image incluse dans la deuxième image frontale, qui est une région d'image dont la position par rapport au tissu de l'oeil du sujet est la même que celle de la région comprenant l'image réfléchie, **caractérisé en ce que**
l'étape de traitement d'image exécute un processus de lissage d'un changement de luminance dans une partie de jonction entre la première image frontale et la deuxième image frontale lorsque l'étape de traitement d'image génère l'image frontale synthétique, et
le dispositif d'imagerie ophtalmique est appliqué à un ophtalmoscope laser à balayage.
